# EUROPEAN PATENT SPECIFICATION

(11) **EP 2 833 929 B1**
(45) Date of publication and mention of the grant of the patent: **27.07.2016**
(21) Application number: 13724402.6
(22) Date of filing: 04.04.2013
(51) Int. Cl.: A61L 15/46, A61L 26/00, A61L 15/42

(54) **ANTIMICROBIAL DRESSING**
ANTIMIKROBIELLER WUNDVERBAND
PANSEMENT ANTIMICROBIEN

(30) Priority: 06.04.2012 IT BO20120185
(43) Date of publication of application: 11.02.2015
(73) Proprietor: Emodial S.r.l., 44100 Cassana (Ferrara) (IT)
(72) Inventor: PECORARI, Gianni, I-41037 Mirandola (Modena) (IT)
(74) Representative: Serravalle, Marco
(86) International application number: PCT/IB2013/052712
(87) International publication number: WO 2013/150487

(56) References cited:
- WO-A1-2008/157092
- WO-A1-2011/019834
- WO-A2-2012/017349
- US-A1- 2011 313 048

## Description

### FIELD OF INVENTION

The invention relates to the field of medical devices, in particular antimicrobial dressings, preferably using catheters.

### DESCRIPTION OF THE PRIOR ART

The skin of a patient can exhibit zones requiring dressing; these zones can comprise cuts, burns, excoriations or wounds. In these cases sterile dressings are used, preferably antimicrobial for preventing proliferation of bacteria. This requirement is particularly felt a the case where a catheter has been implanted in the patient.

Vascular catheters are implanted in a patient when it is necessary to have a vascular access for infusion of liquids and/or administering pharmaceutical substances directly into the vein. This can occur in order to obtain an immediate access in emergency situations and to obtain a permanent vascular access on the patient (for example in oncological situations or peritoneal dialysis). The permanent vascular access is usually constituted by a central venous catheter (CVC), and must be maintained for long periods, and at times can be used for a lifetime.

The implant of a catheter (generic or CVC) on a patient is carried out by inserting a part of the catheter in the body of the patient via a suitable exit site located on the skin. Thus, after the implant, the catheter exhibits a tract internal of the patient and an tract that is external of the body, which can comprise one or more connections for the infusion devices.

To avoid subsequent re-implanting of catheters, the exit point must be closed in the shortest time possible without becoming infected and is periodically dressed with disinfectants and covered with antiseptic dressings to prevent extraluminal catheter contaminations. In particular it is necessary that the exit point stays dry as the presence of liquids (blood or bodily exudates) can facilitate microbial proliferation and increase the risks of infection at the exit point.

This is especially important in the case of a permanent vascular access, especially in a case of patients undergoing chemotherapy or dialytic therapy, for example peritoneal dialysis.

At present, specific antimicrobial dressings are present on the market, under the name of Johnson & Johnson and having the trade name Biopatch, which are conformed as a circular crown to be applied on the skin at the exit point of the catheter so as to surround a section of catheter which exits from the patient's body. These dressings comprise, as an antimicrobial agent, a derivative of chlorhexidine (chlorhexidine digluconate) freeze-dried in a quantity of 10.2 g/m², calculated as a weight of the substance which a fraction of the surface of the dressing applicable on the skin and corresponding to a weight/weight percentage of 2% calculated as a weight of chlorhexidine digluconate in the total weight of the dressing. In the instructions, the dressings must be replaced every seven days.

Although they have a good antimicrobial action, chlorhexidine and the pharmaceutically-acceptable salts (among which digluconate) exhibit a series of potential collateral effects. In fact, experimental studies have shown that they are cytotoxic for fibroblasts; if absorbed they can lead to nephritis and can determine the onset of sensitization or hypersensitisation effects, and irritable skin. The cutaneous sensitization phenomena are particularly undesirable in the case of vascular accesses of the permanent type, such as CVCs in which, in the long-run, these phenomena can give rise to infections. This represents a disadvantage as, in order to be subjected to long and/or periodical intravenous therapies via catheters, the patient has always to maintain an antimicrobial dressing in contact with the skin, and change it periodically.

WO 2008/157092 discloses an anti-microbial dressing comprising a relative portion of dressing with antimicrobial properties having a relative dressing surface, the portion of dressing comprising: chlorhexidine or a pharmaceutically acceptable salt thereof in a solid state in a relative quantity calculated as weight of chlorhexidine or a pharmaceutically acceptable salt 2 thereof /surface of dressing comprised between 2.5 and 50 g/m , and silver sulfadiazine in a solid state in a relative quantity, calculated as weight of silver 2 sulfadiazine /surface of dressing comprised between 0 and 2 g/m².

Further, chlorhexidine and/or the salts thereof, in solutions of above 2% in weight/weight, can cause conjunctivitis or seriously damage the cornea. It follows that it is necessary to take special precautions in the handling of the solutions during the production process of the above-described medications of known type.

There emerges, therefore, the need for an antimicrobial dressing which exceeds the drawbacks of the known dressings and in particular which enables preventing or reducing the replacements of catheters which require unpleasant interventions on the patient.

### SUMMARY OF THE INVENTION

The aim of the present invention is to obviate the drawbacks of the prior art: in particular the main aim of the present invention is to facilitate healing and reduce the probability that the zone to be dressed becomes infected, and at the same time reduces the probability of inflammatory phenomena and/or sensitization and/or irritation of the skin at the zone to be dressed. This, in the case of exit points for catheters, must be both in the post-operations phase and during the permanence of the catheters so as to limit the number of implant procedures a patient has to undergo.

A further aim of the present invention is to provide a dressing usable in particular for dressing the exit points of a catheter in a body, having a high level of antibacterial effectiveness and which substantially reduces the probability of inflammatory phenomena and/or sensitisation and/or irritation of the skin at the dressing zone, when dealing with an exit point of a catheter.

A further aim of the invention is to provide a dressing usable in the case of excoriations and burns of not excessive extent, and can be used to prevent and treat bedsores.

The above aims are obtained in accordance with the independent claim.

In particular, the aims are attained by an antimicrobial medication comprising a relative portion of dressing with antimicrobial properties, having a relative dressing surface, the portion of dressing being characterised by the presence of:
- chlorhexidine and/or a pharmaceutically acceptable salt thereof in a solid state in a relative quantity, calculated as an equivalent weight of chlorhexidine digluconate/surface of dressing, comprised between 0.40 and 1.125 g/m²; and
- silver and/or a pharmaceutically acceptable salt thereof in a solid state in a relative quantity, calculated as an equivalent weight of metallic silver/surface of dressing of greater than or equal to 0.1 mg/m².

The dressing surface is destined to be placed on a zone to be dressed on the patient's skin, preferably at the point of entry of a catheter implanted in a patient.

The dressing of the invention carry out a high level of antimicrobial activity through a wide range. Further, it has surprisingly been found that these dressings s also have a high level of anti-inflammatory activities. This contributes to increasing the antimicrobial effectiveness as the entry point of a catheter is particularly subject to inflammation. The inflammation phenomena at the entry point can subsequently degenerate, giving rise to subsequent inflammations, in particular during the post-operative phase, after the implanting of the catheter.

Further, in a reference study commissioned by the applicant on 128 patients, it was found that a dressing comprising a quantity of metallic silver per unit of surface of the dressing of 0.01 mg/m² renders a high anti-phlogosis effect, thus considerably reducing inflammation at the needle holes made in dialysed patients periodically subjected to venipuncture using the emerging buttonhole technique. This technique includes a first venipuncture with a piercing needle and a series of subsequent venipuncture with a beveled needle. This quantity of silver in the dressing corresponds to a percentage of 0.00016% in weight/weight, considering the specific weight of the dressing used.

Considering that the presence of silver and/or the acceptable pharmaceutical salts thereof enables drastically reducing the quantity of chlorhexidine and/or a pharmaceutically-acceptable salt thereof present in the dressing , the invention enables reducing the risks of sensitisation or hypersensitisation of the entry point and the further counter-indications relating to the presence of chlorhexidine or the salt.

For the purposes of the present invention, the term "dressing" in the following description will be taken to mean those medical devices used for dressings in the form of pads, plasters, compresses, bandages etc.

The term "solid state" is taken to include the solid state in powder form, in particular in nano-particles.

As previously mentioned, the quantity of the antimicrobial agents of the dressing of the invention are determined and expressed respectively as equivalent weight of chlorhexidine diglutonate and metallic silver divided by the surface area of the portion of dressing (in the case of the dressing). Therefore, when the dressing comprises chlorhexidine or another pharmaceutically-acceptable different from chlorhexidine digluconate, it is possible to obtain the relative quantity calculated and expressed as a weight of chlorhexidine digluconate respectively divided by the surface using a factor or conversion given by the relation between the molecular weight of the chlorhexidine diglugonate and the molecular weight of the chlorhexidine derivative effectively comprised in the dressing. Likewise, when the dressing comprises a pharmaceutically acceptable metallic silver salt it is possible to obtain the relative quantity expressed as a weight of metallic silver as a fraction of the surface using a conversion factor given by the relation between the atomic weight of the silver and the molecular of the silver salt effectively comprised in the dressing.

This means that if the dressing comprises another pharmaceutically-acceptable salt of chlorhexidine, for example chlorhexidine diacetate present in the dressing has to be divided by the molecular weight of the chlorhexedine diacetate and multiplied by the molecular weight of the chlorhexidine digluconate before relating it to the surface of the portion of dressing. Likewise, if the dressing comprises a pharmaceutically-accessible salt of silver, for example silver alginate, for the calculation of the respective quantities indicated in the independent claim the weight of the silver alginate must be divided by the molecular weight of the silver alginate and multiplied by the atomic weight of the silver before relating it to the surface of the portion of dressing.

The two antimicrobial agents are in solid form and therefore, once the portion of dressing has been applied on the skin at the exit-site, no liquids are applied to the exit-site which is therefore less subject to microbial growth. Thus, the dressing of the invention is particularly suited to protection of an exit site of a catheter implanted in a patient. For this reason it is particularly preferable to use the dressing for the protection of an exit-site of a catheter implanted on a patient.

Particularly preferred are dressings according to the invention which comprise a least one of the following pharmaceutically-acceptable silver salts; silver nitrate, silver protein (cas number 9008-42-8); silver protein (cas number 9015-51-4), silver alginate, silver sulfadiazine, silver acetate, silver benzoate, silver carbonate, silver iodate, silver iodide, silver lactate, silver laurate, silver oxide, silver palmitate and silver protein, katadyn silver otherwise known as micronized colloidal metallic silver. Most preferred are dressings s according to the invention which comprise metallic silver in particles, preferably in the form of nanoparticles.

Particularly preferred are dressings s according to the invention which comprise at least one from following pharmaceutically-acceptable chlorhexidine salts: chlorhexidine diacetate, chlorhexidine dichlorohydride, chlorhexidine digluconate. Even more preferred are dressings s comprising chlorhexidine digluconate.

The dressing of the invention preferably contains silver or a pharmaceutically-acceptable salt (preferably one of the above-cited salts), preferably in a relative quantity of greater than or equal to 0.2 mg/m², more preferably 0.5 mg/m² more preferably 0.75 mg/m², advantageously 1.0 mg/m². Considering that the increase of this quantity involves a reduction of the quantity of chlorhexidine derivatives, a dressing according to the invention is preferred which contains silver or a pharmaceutically-acceptable salt thereof (preferably one of the above-cited salts) in a relative greater quantity or a same quantity to the following: 0.75 mg/m², 1 mg/m², 5 mg/m², 10 mg/m², 25 mg/m², 50 mg/m², 75 mg/m², mg/m², 100 mg/m², 125 mg/m², 150 mg/m², 175 mg/m², 200 mg/m², 225 mg/m², 250 mg/m², 275 mg/m² e 300 mg/m².

On increasing the above quantity or percentage of silver and/or a pharmaceutically-acceptable salt thereof (calculated as previously indicated) the quantities or percentage of chlorhexidine and or a pharmaceutically-acceptable salt thereof comprised in the dressing and also calculated as previously indicated might fall. Consequently, the disadvantages correlated to these chlorohexidrine-type chemical compounds will fall. It is therefore more greatly preferably for the dressing of the invention to contain chlorhexidine or a pharmaceutically-acceptable salt thereof in a quantity calculated as cited above and indicated in the claims of less than or equal, in the following order, respectively 1.0 g/m², 0.875 g/m², 0.75 g/m² and 0.625 g/m². More preferably, this quantity is greater than or equal to, in the following order, respectively0.425 g/m², 0.450 g/m², 0.475 g/m², 0.500 g/m², 0.525 g/m², 0.550 g/m², 0.575 g/m², 0.600 g/m².

This quantity is comprised between 0.40 and 1.125 g/m², still more advantageously comprised between 0.6 and 0.875 g/m².

The present invention discloses and includes each single combination of the above quantities and/or percentages of the two types of antibacterial substances (i.e. those relative to the chlorhexidine or the salts thereof and the one relating to the silver and its salts/derivatives as indicated above), calculated and expressed as previously indicated and set down in the claims.

Further, also considered to be falling within the ambit of the invention is each single combination of substances at the basis of the chlorhexidine (chlorhexidine or a pharmaceutically-acceptable salt) and silver-based substances (metallic silver or a pharmaceutically-acceptable salt) in particular comprising one or more of the substantially specially mentioned herein above. Finally, the scope of the present description also includes and comprises each single combination of the above combinations of quantity and/or percentages and the cited combinations of substances.

Also preferred is a dressing according to the invention, in which the quantity of the silver and/or a pharmaceutically-acceptable salt in the solid state, calculated as an equivalent weight of metallic silver/surface of dressing, is greater than 0.2 mg/m².

Especially preferred are dressings according to the invention, in which the portion of dressing has a ratio between the weight thereof and the dressing surface comprised between 100 and 150 g/m², preferably between 120 - 130 g/m², and comprises:
the chlorhexidine and/or a pharmaceutically acceptable salt thereof in the solid state in a relative quantity, calculated as an equivalent weight of chlorhexidine digluconate/surface of medication, is comprised between 0.500 and 0.875 g/m² and corresponding to a relative percentage, calculated as an equivalent weight of chlorhexidine digluconate/weight of the portion (30) of dressing in percentage, comprised between 0.40 and 0.70%;
and the silver and/or a pharmaceutically acceptable salt thereof in the solid state in a relative quantity, calculated as an equivalent weight of metallic sliver/surface of medication, is comprised between 50 and 300 mg/m² (preferably between 50 and 250 mg/m², more preferably between 50 and 200 mg/m²) and corresponding to a relative percentage, calculated as an equivalent weight of metallic silver/weight of the portion of dressing in percentage, comprised between 0.04% and 0.24% (preferably between 0.04 % and 0.16%, more preferably 0.20%).

### BRIEF DESCRIPTION OF THE DRAWINGS

The characteristics of the invention will emerge in the following, in which some preferred but not exclusive embodiments are described with reference to the accompanying tables of drawings, in which:
figure 1 is a perspective and schematic view, not in scale, of an embodiment of the dressing of the invention;
figure 2 is a perspective and schematic view, not in scale, of an embodiment of the dressing of the invention;
figure 3 is a perspective view, not in scale, of a further embodiment of the dressing of the invention;
figure 3a is an embodiment of the dressing in an alternative confirmation to the one shown in the embodiment of the invention of figure 3;
figure 4 is a perspective view of a further embodiment of the dressing of the invention;
figure 5 is a perspective view of a further embodiment of the dressing of the invention.

### DESCRIPTION OF PREFERRED EMBODIMENTS

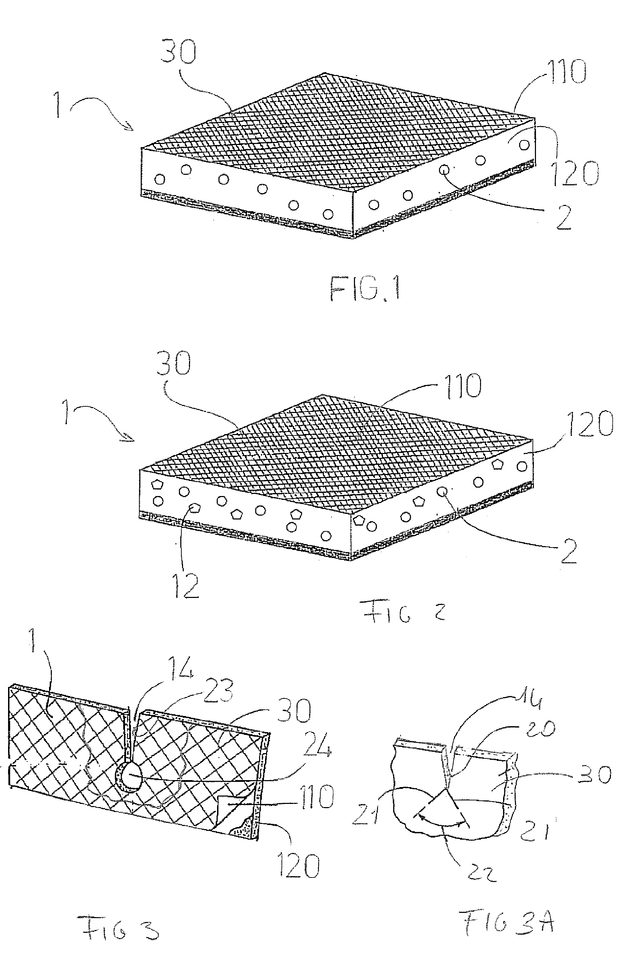

In the various figures 1-5, the identical components are denoted by the same numerical references as in the following description in the various embodiments of the invention and have similar characteristics unless otherwise specified.

In the figures, 1 denotes a dressing comprising a relative portion of dressing 30 with antimicrobial properties having a relative surface of dressing 110 in which the portion of dressing 30 comprises:
chlorhexidine and/or a pharmaceutically acceptable salt thereof in a solid state in a relative quantity, calculated as an equivalent weight of chlorhexidine digluconate/surface of dressing, comprised between 0.40 and 1,125 g/m²; and
silver and/or a pharmaceutically acceptable salt thereof in a solid state in a relative quantity, calculated as an equivalent weight of metallic silver/surface of dressing of greater than or equal to 0.1 mg/m².

In the case of a dressing 1 for protection of an entry point of a catheter implanted in a patient, the relative portion of dressing 30 is preferably conformed in such a way as to surround the catheter at a relative section external of the body of the patient when the surface of the dressing 110 is applied on the skin at the exit point (figures 3-4).

In particular, a dressing 1 is preferred for protection of a catheter implanted in a patient in which the relative portion of dressing 30 is interested by a slit 14 having a shape and dimensions that are such as to enable a tract of catheter external of the body to cross the portion 30 of dressing and surround the sector of the catheter when the medicating surface is applied at the grafting exit point. Alternatively the health operative and/or the patient can form a slit 14 in the dressings 1 which are lacking at moment of use, by using sterile cutting means. This can be performed, for example, using the dressings 1 illustrated in figures 1-2.

As illustrated in figures 3-4, the slit 14 is preferably selected from a group comprising:
a single cut comprising a single linear cut 23 having a relative first end arranged on an edge 35 of the portion of dressing 30; and a through-hole 20 which originates from a second end of the single cut 23;
a Y-shaped slit comprising a first linear cut 20 having a relative first end arranged on an edge 35 of the portion of dressing 30; and two further linear cuts 21 originating from a second of the first cut 20 and identifying an angle 22 of less than 180°.

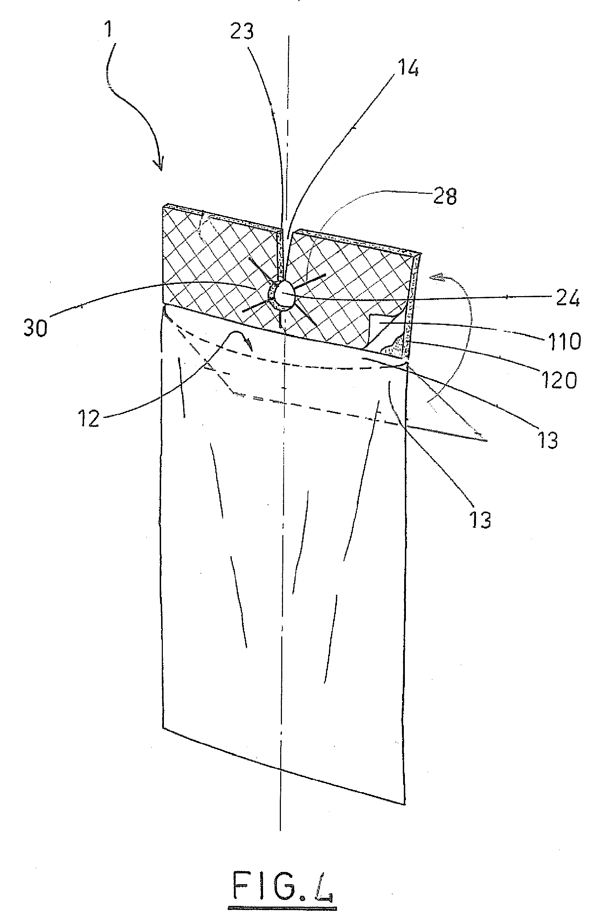

The through-hole 24 is preferably circular and the slit 14 further comprises at least a plurality of further cuts 28 and wherein each of the further cuts 28 originates from the edge of the through-hole 24 and is arranged, externally of the through-hole 24, along a radial direction with respect to the through-hole 24 (figure 4).

The dressings 1 are particularly preferred for protecting an exit point of a catheter implanted in a patient according the invention as illustrated in figure 4. They are provided with the slit 14, and further comprise a containing element 10 for containing the tract of catheter which remains external of the patient's body once the catheter has been installed. This containing element 10 in turn comprises: a housing (preferably defined by two facing walls 13) having dimensions that are such as to be able to receive a terminal part of the external tract of catheter; and an opening 12 for placing the housing in communication with the outside. The opening 12 is conformed and arranged in such a way as to enable introduction and positioning of the terminal part of the external tract of the catheter in the housing when the dressing surface of the portion of dressing is applied on the patient's body at an exit point and the slit surrounding the external tract of catheter. Therefore the containing element 10 is fixed to the portion of dressing in such a way that the opening 12 is arranged on the opposite side to the dressing surface. The dressing portion 30 performing an antimicrobial activity is preferably arranged in proximity of the opening 12, more preferably contiguous to the opening 12.

In an embodiment of the dressing 1 according to the invention (not illustrated) the relative dressing portion 30 comprises an element made of a metallic silver which can be constituted by a slim pierced layer of metallic silver, by a netting or by a mesh realized with skeins of metallic silver. In this embodiment the chlorhexidine and/or the pharmaceutically-acceptable salt thereof is arranged on the element made of metallic silver. The metallic silver element is permeable to blood and to bodily exudate and is preferably arranged on a plaster.

In an embodiment of the dressing 1 of the invention, not illustrated, the relative portion of dressing 30 comprises a metallic silver element which can be constituted by a slim pierced layer of metallic silver, a net or a mesh realized with threads of metallic silver. In this embodiment the chlorhexidine and/or the pharmaceutically-acceptable salt is arranged on the metallic silver element. The metallic silver element is permeable to blood and to bodily exudate and is preferably arranged on a plaster.

Figures 1, 2, 3, 4 and 5 illustrate dressings 1 in which the relative portion of dressing 30 further comprises:
a surface layer 110 identifying the surface of the dressing for contacting a patient's skin, the layer being permeable to blood and corporeal exudate; and at least an absorbent inner layer 120 for absorbing blood and corporeal exudate;
wherein at least one from among the surface layer 110 and/or the internal absorbent layer 120 comprises the chlorhexidin and/or the pharmaceutically acceptable salt thereof and the silver and/or the pharmaceutically acceptable salt thereof.

As in figure 1, the surface layer 110 advantageously comprises metallic silver and the internal layer comprises chlorhexidin or a salt thereof (schematized and illustrated as particles denoted by reference numeral 2).

The internal absorbent layer 120 preferably comprises the chlorhexidine and/or the pharmaceutically-acceptable salt.

The surface layer 110 is preferably obtained by metallization with silver of an anti-stick support (not shown) which is permeable to blood and corporeal exudate which can be a mesh, a textile, a TNT, made of polyethylene, polyester, viscose, or other medical-grade material. The metallization can be done using classical methods or by micro-deposition or nano-deposition.

As in figure 1, the surface layer 110 advantageously comprises metallic silver and the internal layer comprises chlorhexidine or a salt thereof schematised and illustrated as particles denoted by reference 2. In figures 1, 2,4 and 5, the surface layer 110 is constituted by a metallic silver element, in particular by a net realized with threads of metallic silver.

Figure 2 illustrates an embodiment of the dressing in which the silver nano-particles and/or a pharmaceutically-acceptable salt thereof are schematized by reference numeral 12.

The dressings 1 s of the invention comprising also nano-particles 2 of chlorhexidine or a pharmaceutically-acceptable salt thereof are preferably (figure 2).

Therefore the dressings of the invention are preferred in which the chlorhexidine and/or a pharmaceutically-acceptable salt thereof and/or the silver and/or a pharmaceuctically-acceptable salt thereof are in the form of nano-particles 2, 12, in particular when each single nano-particle 2, 12 comprises both the chlorhexidine and/or a pharmaceutically-acceptable salt thereof is silver and/or a pharmaceutically-acceptable salt thereof (figure 2).

These bicomponent nanoparticles, comprising silver, preferably metallic, and/or a pharmaceutically-acceptable salt and chlorohexidrin and/or a pharmaceutically-acceptable salt thereof, are obtainable by means of contemporary nano-deposition of the substances. In the case of the dressing 1 according to the invention, the nano-particles 1 can be deposited on a suitable sub-layer by means of known methods, including successive depositing of silver nanoparticles, preferable metallic silver, and nanoparticles of chlorhexidine and/or a relative pharmaceutically-acceptable salt or vice versa.

The sub-layer on which the nano-particles 2, 12 are deposited can be advantageously constituted by the surface layer 110 and/or by the absorbent layer 120 should the dressings 1 require the presence thereof.

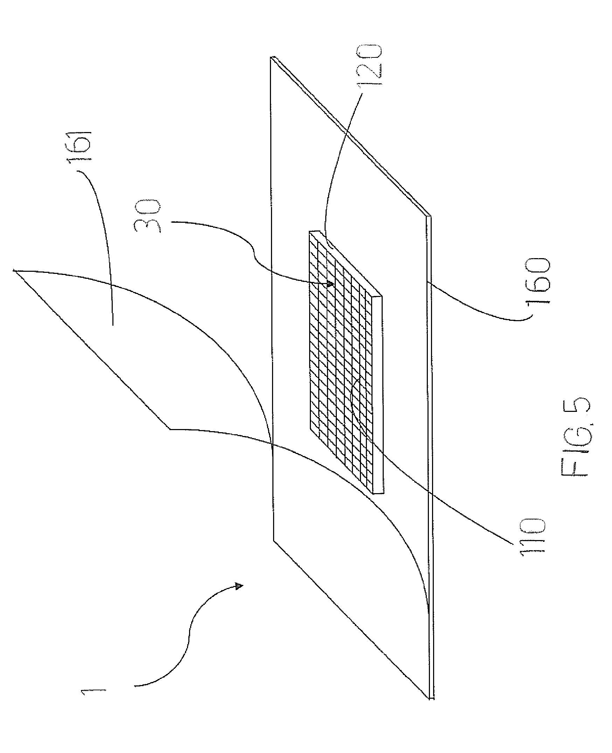

Also particularly advantageous are the embodiments of the dressing that comprise a plaster, an elastic bandage, a self-adhesive bandage, as they enable easily fixing the dressing surface to the patient's skin. Figure 5 illustrates an embodiment of the dressing further comprising a plaster constituted by an adhesive layer 160 on which the portion of dressing is arranged so as to fix the portion to the zone to be dressed and by at least a removably protective film 161. For the sake of greater clarity, in figure 5 the flaps of the two parts of the protective film 161 have been illustrated in a raised position, instead of completely opposite the adhesive layer and the portion of dressing.

The portion of dressing, generally planar, can have any shape: circular (as in figure 5), square, rectangular, etc.

Obviously the dressings of the invention will have to be sterilized before use, preferably before the relative packing.

### Experimental data

The antimicrobial activity was tested following the ASTM E 2180-07 method, on 90 samples of a sterile dressing according to the invention which differed from the one in figure 1 only in that it was circular and not rectangular. The dressing comprises chlorhexidine digluconate 0.625 g/m²; and metallic silver 200 mg/m², and the silver is metallized on the surface layer 110 while the chlorhexidine digluconate is arranged in the absorbent layer 120. As control, 90 samples of a sterile dressing was used, which differed from the dressing of the invention only in the absence of metallic silver and chlorhexidine digluconate.

Four microbial strains were tested, as follow: Candida albicans ATCC 10231; Pseudomonas aeruginosa ATCC 15442; Staphylococcus aureus ATCC 6538 Staphylococcus aureus MRSA ATCC33591.

The results obtained have been summarized in the following tables.

**Table 2: strain count (ufc/ml)**

| Strains | Dilution 10⁻⁶ (ufc/ml) | Result (ufc/ml) | Inoculum (ufc/ml) |
|---|---|---|---|
| Candida albicans ATCC 10231 | 203 - 240 | 2.22 x 10⁸ | 2.22 x 10⁶ |
| Pseudomonas aeruginosa ATCC 15442 | 116 - 101 | 1.09 x 10⁸ | 1.09 x 10⁶ |
| Staphylococcus aureus ATCC 6538 | 149 - 153 | 1.51 x 10⁸ | 1.51 x 10⁶ |
| Staphylococcus aureus MRSA ATCC33591 | 133 - 151 | 1.42 x 10⁸ | 1.42 x 10⁶ |

**Table 3: mean count of surviving micro-organisms (ufc/ml) at different contact times (hours)**

| Strain | times | T 0 | T 24 hours | T 48 hours | T 72 hours | T 120 hours | T 144 hours | T 168 hours | T 192 hours |
|---|---|---|---|---|---|---|---|---|---|
| Candida albicans | control | 4.47 x 10⁵ | 2.09 x 10⁵ | 2.19 x 10⁵ | 3.02 x 10⁵ | 4.27 x 10⁵ | 5.50 x 10⁵ | 1.51 x 10⁵ | N.E. |
| ATCC 10231 | sample | 1.45 x 10⁵ | < 100 | <10 | < 10 | <10 | < 10 | < 10 | N.E. |
| Pseudomonas aeruginosa ATCC 15442 | control | 1.02 x 10⁵ | 9.33 x 10⁵ | N.E. | 9.55 x 10⁵ | 1.20 x 10⁶ | 1.45 x 10⁶ | 1.23 x 10⁶ | N.E. |
| | sample | 8.5 x 10⁴ | 166 | N.E. | < 10 | <10 | < 10 | < 10 | N.E. |
| Staphylococ cus aureus ATCC 6538 | control | 8.9 x 10⁴ | 1.55 x 10⁵ | 1.66 x 10⁵ | 1.70 x 10⁵ | 2.00 x 10⁵ | 2.00 x 10⁵ | 2.19 x 10⁵ | 2.04 x 10⁵ |
| | sample | 2.14 x 10⁴ | <10 | <10 | < 10 | <10 | < 10 | < 10 | <10 |
| Staphylococcus aureus MRSA ATCC33591 | control | 1.78 x 10⁵ | 1.70 x 10⁵ | 1.82 x 10⁵ | 2.14 x 10⁵ | 2.29 x 10⁵ | 2.51 x 10⁵ | 2.40 x 10⁵ | 2.69 x 10⁵ |
| | sample | 2.95 x 10⁴ | 269 | 37 | <10 | <10 | < 10 | < 10 | <10 |

| | | | | | | | | | |
|---|---|---|---|---|---|---|---|---|---|
| N.E. = analysis not performed. | | | | | | | | | |

**Table 4 : mean logarithmic expression of surviving micro-organisms 5 at different contact times**

| Strain | time | T 0 | T 24 hours | T 48 hours | T 72 hours | T 120 hours | T 144 hours | T 168 hours | T 192 hours |
|---|---|---|---|---|---|---|---|---|---|
| Candida albicans ATCC 10231 | control | 5.65 | 5.32 | 5.34 | 5.48 | 5.63 | 5.74 | 5.18 | N.E. |
| | sample | 5.16 | < 10.00 | < 1.00 | < 1.00 | < 1.00 | < 1.00 | < 1.00 | N.E. |
| Pseudomonas aeruginosa ATCC 15442 | Control | 5.01 | 5.97 | N.E. | 5.98 | 6.08 | 6.16 | 6.09 | N.E. |
| | Sample | 4.93 | 2.22 | N.E. | < 1.00 | < 1.00 | < 1.00 | < 1.00 | N.E. |
| Staphylococcus aureus ATCC 6538 | Control | 4.95 | 5.19 | 5.22 | 5.23 | 5.30 | 5.30 | 5.34 | 5.31 |
| | Sample | 4.33 | < 1.00 | < 1.00 | < 1.00 | < 1.00 | < 1.00 | < 1.00 | < 1.00 |
| Staphylococcus aureus MRSA ATCC33591 | Control | 5.25 | 5.23 | 5.26 | 5.33 | 5.36 | 5.40 | 5.38 | 5.43 |
| | Sample | 4.47 | 2.43 | 1.57 | < 1.00 | < 1.00 | < 1.00 | < 1.00 | < 1.00 |

| | | | | | | | | | |
|---|---|---|---|---|---|---|---|---|---|
| N.E. = analysis not performed. | | | | | | | | | |

**Table 5: percentage reduction at different contact times**

| Strains | T 24 (hours) | T 48 (hours) | T 72 (hours) | T 120 (hours) | T 144 (hours) | T 168 (hours) | T 192 (hours) |
|---|---|---|---|---|---|---|---|
| Candida albicans ATCC 10231 | 99.95% | 99.99% | 99.99% | 99.99% | 99.99 % | 99.99 % | N.E. |
| Pseudomonas aeruginosa ATCC 15442 | 99.98% | N.E. | 99.99% | 99.99% | 99.99 % | 99.99 % | N.E. |
| Staphylococcus aureus ATCC 6538 | 99.99% | 99.99% | 99.99% | 99.99% | 99.99 % | 99.99 % | 99.99 % |
| Staphylococcus aureus MRSA ATCC33591 | 99.84% | 99.98% | 99.99% | 99.99% | 99.99 % | 99.99 % | 99.99 % |

| | | | | | | | |
|---|---|---|---|---|---|---|---|
| N.E. = analysis not performed. | | | | | | | |

### Conclusions

On the basis of the results obtained, and respecting the testing validity criteria, the tested samples of the dressings of the invention comprising chlorhexidin digluconate 0.625 g/m² and metallic silver 200 mg/m², in the experimental conditions used, are effective as antimicrobials in the comparisons of the tested strains, up to 168 hours (7 days) [192 hours - 8 days - for the staphylococcus strains], in accordance with what is set by standard ASTM E-2180-07. Therefore it has been demonstrated that the dressing of the invention can be used for 7 days (168 hours), like the prior art, notwithstanding the fact it comprises a quantity of chlorhexidin digluconate that is significantly lower, i.e. 0.625 g/m² instead of the quantity used in the known dressing, 10.2 g/m². This enables considerably reducing the disadvantages correlated to the chlorhexidine digluconate.

As can be gleaned from the table, it can be deduced that the analysed dressings can carry out an antibacterial activity even for time periods greater than 7 days (192 hours).

Further, the results obtained confirm that the dressing of the invention can be advantageously used in the case of excoriations and burns not excessively widespread, often with the risk of infection on the part of the microbial strains for which the antimicrobial effectiveness has been tested.

Similar and further advantages can be exhibited for the prevention and treatment of bedsores.

## Claims

1. An anti-microbial dressing comprising a relative portion (30) of dressing with antimicrobial properties having a relative dressing surface (110), the portion (30) of dressing comprising: chlorhexidine and/or a pharmaceutically acceptable salt thereof in a solid state in a relative quantity, calculated as an equivalent weight of chlorhexidine digluconate/surface of dressing, comprised between 0.40 and 1.125 g/m², and silver and/or a pharmaceutically acceptable salt thereof in a solid state in a relative quantity, calculated as an equivalent weight of metallic silver/surface of dressing of greater than or equal to 0.1 mg/m².

2. The dressing of the preceding claim, wherein the quantity of the chlorhexidine and/or a pharmaceutically acceptable salt thereof in solid state, calculated as an equivalent weight of chlorhexidine digluconate/surface of dressing, is greater than or equal to 0.425 mg/m².

3. The dressing of any one of the preceding claims, wherein the quantity of the silver and/or a pharmaceutically acceptable salt thereof in solid state in a relative quantity, calculated as an equivalent weight of metallic silver/surface of dressing, is greater than or equal to 0.2 mg/m².

4. The dressing of any one of the preceding claims, wherein the portion (30) of dressing has a ratio between the weight thereof and the surface of the dressing that is comprised between 100 and 150 g/m², and comprises: the chlorhexidine and/or a pharmaceutically acceptable salt thereof in the solid state in a relative quantity, calculated as an equivalent weight of chlorhexidine digluconate/surface of medication, is comprised between 0.500 and 0.875 g/m² and corresponding to a relative percentage, calculated as an equivalent weight of chlorhexidine digluconate/weight of the portion (30) of dressing in percentage, comprised between 0.40 and 0.70%; and the silver and/or pharmaceutically acceptable salt thereof in the solid state in a relative quantity, calculated as an equivalent weight of metallic sliver/surface of medication, is comprised between 50 and 300 mg/m² and corresponding to a relative percentage, calculated as an equivalent weight of metallic silver/weight of the portion of dressing in percentage, comprised between 0.04% and 0.24%.

5. The dressing of any one of the preceding claims, wherein the chlorohexidine and/or a pharmaceutically acceptable salt thereof and/or the silver and/or a pharmaceutically acceptable salt thereof are in a nanoparticle form (2, 12).

6. The dressing of the preceding claim, wherein each single nanoparticle (2, 12) comprises both the chlorohexidin and/or a pharmaceutically acceptable salt thereof and the silver and/or a pharmaceutically acceptable salt thereof.

7. The dressing of any one of the preceding claims, wherein the portion (30) of dressing comprises a metallic silver element selected from a group constituted by: a net or a mesh realised using wires of metallic silver and a slim perforated layer of metallic silver; and wherein the chlorhexidine and/or the pharmaceutically acceptable salt thereof is arranged on the metal element.

8. The dressing of any one of the preceding claims from 1 to 6, wherein the relative portion (30) of dressing further comprises:
a surface layer (110) identifying the surface of the dressing for contacting a patient's skin, the layer being permeable to blood and corporeal exudate; and
at least an absorbent inner layer (120) for absorbing blood and corporeal exudate; wherein at least one from among the surface layer (110) and/or the internal absorbent layer (120) comprises the chlorhexidine and/or the pharmaceutically acceptable salt thereof and the silver and/or the pharmaceutically acceptable salt thereof.

9. The dressing of any one of the preceding claims for protecting an grafting exit point of a catheter implanted in a patient, in which the relative portion (30) of dressing is conformed in such a way as to surround the catheter at a sector of the catheter external of the patient's body when the medicating surface (110) is applied on the skin at the grafting exit point.

10. The dressing of the preceding claim, wherein the relative portion (30) of dressing is interested by a slit (14) having a shape and dimensions that are such as to enable a tract of catheter external of the body to cross the portion (30) of dressing and surround the sector of the catheter when the medicating surface (110) is applied at the grafting exit point.

## Patentansprüche

1. Antimikrobieller Verband, umfassend einen relativen Teil (30) an Verband mit antimikrobiellen Eigenschaften mit einer relativen Verbandsoberfläche (110), wobei der Teil (30) an Verband Folgendes umfasst: Chlorhexidin und/oder ein pharmazeutisch annehmbares Salz davon in einem festen Zustand in einer relativen Menge, berechnet als ein äquivalentes Gewicht an Chlorhexidindigluconat/Oberfläche an Verband, umfassend zwischen 0,40 und 1,125 g/m², und Silber und/oder ein pharmazeutisch annehmbares Salz davon in einem festen Zustand in einer relativen Menge, berechnet als ein äquivalentes Gewicht an metallischem Silber/Oberfläche an Verband von mehr als oder gleich 0,1 mg/m².

2. Verband nach dem vorhergehenden Anspruch, wobei die Menge des Chlorhexidins und/oder eines pharmazeutisch annehmbaren Salzes davon in festem Zustand, berechnet als ein äquivalentes Gewicht an Chlorhexidindigluconat/Oberfläche an Verband mehr als oder gleich 0,425 mg/m² ist.

3. Verband nach einem der vorhergehenden Ansprüche, wobei die Menge an dem Silber und/oder eines pharmazeutisch annehmbaren Salzes davon in festem Zustand in einer relativen Menge, berechnet als ein äquivalentes Gewicht an metallischem Silber/Oberfläche an Verband mehr als oder gleich 0,2 mg/m² ist.

4. Verband nach einem der vorhergehenden Ansprüche, wobei der Teil (30) an Verband ein Verhältnis zwischen dem Gewicht davon und der Oberfläche des Verbands umfassend zwischen 100 und 150 g/m² aufweist, und Folgendes umfasst: das Chlorhexidin und/oder ein pharmazeutisch annehmbares Salz davon in dem festen Zustand in einer relativen Menge, berechnet als ein äquivalentes Gewicht an Chlorhexidindigluconat/Oberfläche an Medikament, zwischen 0,500 und 0,875 g/m² umfassend und einem relativen Prozentsatz entsprechend, berechnet als ein äquivalentes Gewicht an Chlorhexidindigluconat/Gewicht des Teils (30) an Verband als Prozentsatz, umfassend zwischen 0,40 und 0,70 %; und das Silber und/oder pharmazeutisch annehmbare Salz davon in dem festen Zustand in einer relativen Menge, berechnet als ein äquivalentes Gewicht an metallischem Silber/Oberfläche an Medikament, zwischen 50 und 300 mg/m² umfassend und einem relativen Prozentsatz entsprechend, berechnet als ein äquivalentes Gewicht an metallischem Silber/Gewicht des Teils des Verbands als Prozentsatz, umfassend zwischen 0,04 % und 0,24 %.

5. Verband nach einem der vorhergehenden Ansprüche, wobei das Chlorhexidin und/oder ein pharmazeutisch annehmbares Salz davon und/oder das Silber und/oder ein pharmazeutisch annehmbares Salz davon in einer Nanopartikelform (2, 12) sind.

6. Verband nach dem vorhergehenden Anspruch, wobei jedes einzelne Nanopartikel (2, 12) sowohl das Chlorhexidin und/oder ein pharmazeutisch annehmbares Salz davon als auch das Silber und/oder ein pharmazeutisch annehmbares Salz davon umfasst.

7. Verband nach einem der vorhergehenden Ansprüche, wobei der Teil (30) an Verband ein metallisches Silberelement umfasst, das aus einer Gruppe ausgewählt ist, die gebildet wird durch: ein Netz oder ein Geflecht, das unter Verwendung von Drähten aus metallischem Silber und einer dünnen perforierten Schicht an metallischem Silber realisiert ist, und wobei das Chlorhexidin und/oder das pharmazeutisch annehmbare Salz davon an dem Metallelement angeordnet ist.

8. Verband nach einem der vorhergehenden Ansprüche 1 bis 6, wobei der relative Teil (30) an Verband weiter Folgendes umfasst:
eine Oberflächenschicht (110), die die Oberfläche des Verbands zum Kontaktieren der Haut eines Patienten identifiziert, wobei die Schicht blut- und körperexsudatsdurchlässig ist; und
mindestens eine absorbierende innere Schicht (120) zum Absorbieren von Blut und Körperexsudat; wobei mindestens eine von der Oberflächenschicht (110) und/oder der inneren absorbierenden Schicht (120) das Chlorhexidin und/oder das pharmazeutisch annehmbare Salz davon und das Silber und/oder das pharmazeutisch annehmbare Salz davon umfasst.

9. Verband nach einem der vorhergehenden Ansprüche zum Schützen eines Transplantationsaustrittspunktes eines in einem Patienten implantierten Katheters, wobei der relative Teil (30) an Verband auf eine solche Weise angepasst ist, dass er den Katheter in einem Bereich des Katheters außerhalb des Patientenkörpers umgibt, wenn die Medikationsoberfläche (110) an dem Transplantationsaustrittspunkt auf der Haut angewandt wird.

10. Verband nach dem vorhergehenden Anspruch, wobei der relative Teil (30) an Verband durch einen Schlitz (14) mit einer Form und Maßen, die derart sind, dass sie ermöglichen, dass ein Teil des Katheters außerhalb des Körpers den Teil (30) an Verband durchläuft und den Bereich des Katheters umgibt, wenn die Medikationsoberfläche (110) auf dem Transplantationsaustrittspunkt angewandt wird, interessiert wird.

## Revendications

1. Pansement antimicrobien comprenant une partie relative (30) de pansement avec des propriétés antimicrobiennes ayant une surface de pansement relative (110), la partie (30) de pansement comprenant : de la chlorhexidine et/ou un sel pharmaceutiquement acceptable de celle-ci dans un état solide dans une quantité relative, calculée comme poids équivalent de digluconate de chlorhexidine/surface de pansement, comprise entre 0,40 et 1,125 g/m², et de l'argent et/ou un sel pharmaceutiquement acceptable de celui-ci dans un état solide dans une quantité relative, calculée comme poids équivalent d'argent métallique/surface de pansement, supérieure ou égale à 0,1 mg/m².

2. Pansement selon la revendication précédente, dans lequel la quantité de la chlorhexidine et/ou d'un sel pharmaceutiquement acceptable de celle-ci dans un état solide, calculée comme poids équivalent de digluconate de chlorhexidine/surface de pansement, est supérieure ou égale à 0,425 mg/m².

3. Pansement selon l'une quelconque des revendications précédentes, dans lequel la quantité de l'argent et/ou d'un sel pharmaceutiquement acceptable de celui-ci dans un état solide dans une quantité relative, calculée comme poids équivalent d'argent métallique/surface de pansement, est supérieure ou égale à 0,2 mg/m².

4. Pansement selon l'une quelconque des revendications précédentes, dans lequel la partie (30) de pansement a un rapport entre le poids de celle-ci et la surface du pansement qui est compris entre 100 et 150 g/m², et comprend : la chlorhexidine et/ou un sel pharmaceutiquement acceptable de celle-ci dans l'état solide dans une quantité relative, calculée comme poids équivalent de digluconate de chlorhexidine/surface de médication, qui est comprise entre 0,500 et 0,875 g/m² et correspondant à un pourcentage relatif, calculé comme poids équivalent de digluconate de chlorhexidine/poids de la partie (30) de pansement en pourcentage, compris entre 0,40 et 0,70 % ; ; et l'argent et/ou un sel pharmaceutiquement acceptable de celui-ci dans l'état solide dans une quantité relative, calculée comme poids équivalent d'argent métallique/surface de médication, qui est comprise entre 50 et 300 mg/m² et correspondant à un pourcentage relatif, calculé comme poids équivalent d'argent métallique/poids de la partie de pansement en pourcentage, compris entre 0,04 % et 0,24 %.

5. Pansement selon l'une quelconque des revendications précédentes, dans lequel la chlorhexidine et/ou un sel pharmaceutiquement acceptable de celle-ci et/ou l'argent et/ou un sel pharmaceutiquement acceptable de celui-ci sont dans une forme nanoparticulaire (2, 12).

6. Pansement selon la revendication précédente, dans lequel chaque nanoparticule individuelle (2, 12) comprend à la fois la chlorhexidine et/ou un sel pharmaceutiquement acceptable de celle-ci et l'argent et/ou un sel pharmaceutiquement acceptable de celui-ci.

7. Pansement selon l'une quelconque des revendications précédentes, dans lequel la partie (30) de pansement comprend un élément d'argent métallique choisi dans un groupe constitué par : un filet ou une maille réalisé à l'aide de fils d'argent métallique et une couche perforée mince d'argent métallique ; et dans lequel la chlorhexidine et/ou le sel pharmaceutiquement acceptable de celle-ci sont disposés sur l'élément métallique.

8. Pansement selon l'une quelconque des revendications précédentes de 1 à 6, dans lequel la partie relative (30) de pansement comprend de plus :
une couche de surface (110) identifiant la surface du pansement pour venir en contact avec la peau d'un patient, la couche étant perméable au sang et à l'exsudat corporel ; et
au moins une couche interne absorbante (120) pour absorber le sang et l'exsudat corporel ; au moins l'une parmi la couche de surface (110) et/ou la couche absorbante interne (120) comprenant la chlorhexidine et/ou le sel pharmaceutiquement acceptable de celle-ci et l'argent et/ou le sel pharmaceutiquement acceptable de celui-ci.

9. Pansement selon l'une quelconque des revendications précédentes pour protéger un point de sortie de greffage d'un cathéter implanté dans un patient, dans lequel la partie relative (30) de pansement est conformée d'une manière qu'elle entoure le cathéter à un secteur du cathéter extérieur au corps du patient lorsque la surface de médication (110) est appliquée sur la peau au point de sortie de greffage.

10. Pansement selon la revendication précédente, dans lequel la partie relative (30) du pansement comporte une fente (14) ayant une forme et des dimensions qui sont telles qu'elles permettent à une étendue de cathéter extérieure au corps de traverser la partie (30) de pansement et d'entourer le secteur du cathéter lorsque la surface de médication (110) est appliquée au point de sortie de greffage.
